# EUROPEAN PATENT APPLICATION

(11) **EP 3 838 267 A1**
(43) Date of publication of application: **23.06.2021**
(21) Application number: 19383144.3
(22) Date of filing: 19.12.2019
(51) Int. Cl.: A61K 9/20, A61K 31/444

(54) **EDOXABAN TABLETS**

(71) Applicant: BIOHORM, S.L., 08184 Palau Solita i Plegamans (Barcelona) (ES)
(72) Inventor: STRUSI, Orazio Luca, 08206 Sabadell - Barcelona (ES); HERNÁNDEZ BALLESTER, José Vicente, 08203 Sabadell - Barcelona (ES)
(74) Representative: ABG Intellectual Property Law, S.L.

(57) **Abstract**

The present invention relates to solid pharmaceutical formulations comprising edoxaban as an active ingredient, namely tablets comprising edoxaban, a first diluent and a second diluent in its composition having good dissolution properties, and to a process for producing the same.

## Description

### FIELD OF THE INVENTION

The present invention relates to solid pharmaceutical formulations comprising edoxaban as an active ingredient, in particular relating to tablets for oral administration, and to a process to prepare the same.

### BACKGROUND OF THE INVENTION

Edoxaban is the common name of the chemical compound N'-(5-chloropyridin-2-yl)-*N*-[(1*S*,2*R*,4*S*)-4-(dimethylcarbamoyl)-2-[(5-methyl-6,7-dihydro-4*H*-[1,3]thiazolo [5,4-c]pyridine-2-carbonyl)amino]cyclohexyl]oxamide, and has the chemical structure shown below.

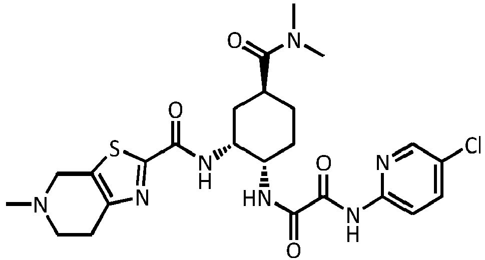

It is an anti-coagulant drug, namely a direct factor Xa inhibitor, a class of anti-coagulants comprising other drugs ("xabans") such as rivaroxaban, apixaban and betrixaban, which was developed as an alternative to classic anti-coagulants such as heparin(s) and warfarin, due to the limitations in administration routes (heparins are injectable), and/or narrow therapeutic window (in the case of warfarin).

This compound is disclosed in patent document EP 1 405 852 A1, and was developed by Daiichi Sankyo as a fast-acting, selective and reversible inhibitor of factor Xa, which can be orally administrated in a once-daily regimen. It is commercially available and marketed in Europe, under the name Lixiana®, in the form of the corresponding tosylate salt (edoxaban tosylate).

Edoxaban is a basic compound, and thus can be solubilized in strong acidic aqueous solution, however when the pH of the aqueous media is shifted to less acidic, neutral or basic values, the dissolution profile of edoxaban diminishes considerably.

Several documents are known in the art dealing with improving the dissolution properties of edoxaban and corresponding pharmaceutical compositions. For instance, patent document EP 2 140 867 A1, discloses oral solid dosage forms, namely tablets coated or not, comprising edoxaban together with a sugar alcohol (mannitol) and/or a water-swelling additive, and other excipients. However, use of sugar alcohols may be problematic in patients showing intolerance against these compounds, such as mannitol. Furthermore, document EP 2 140 867 A1 discloses that compositions where the sugar alcohol is replaced by other excipients, such as a combination of a saccharide (lactose), and a starch (cornstarch) the dissolution profile of the resulting compositions at pH 4 (strong acidic medium) is not satisfactory.

Patent document EP 2 548 556 A1, discloses that keeping the water content during granulation of edoxaban-containing material to 10% or less, leads to an improved dissolution profile in the neutral region of the pH scale. However, this improvement is only obtained in formulations having a sugar alcohol in its composition. As mentioned above, many patients show intolerance against sugar alcohols, such as mannitol. Yet, it is further known that these excipients also increase the gastrointestinal motility, leading to a decrease in drug absorption, which involves the risk of lower efficacy of the therapeutic ingredient.

Patent document EP 3 177 290 A1, discloses tablet compositions comprising edoxaban in combination with a water-soluble vinylpyrrolidone polymer (copovidone, povidone) and a cellulose ether, free of a sugar alcohol. However, no experimental data is provided detailing the dissolution profile of said compositions, thus there is no information on the influence of the disclosed compositions in the dissolution of edoxaban at any pH value. Furthermore, the use of a water-soluble vinylpyrrolidone polymer affects the stability of the tablets. Because of its high peroxide content, vinylpyrrolidone polymers tend to oxidatively degrade the active ingredient and the excipients causing lower tablet stability, which results eventually in lower therapeutic efficacy.

As can be seen there is still a need for new oral dosage forms, tablets, with an adequate dissolution profile, which are free of sugar alcohols in its composition. Therefore, it is an object of this invention to provide a tablet formulation comprising edoxaban or a pharmaceutically acceptable salt thereof, having no sugar alcohols in its composition, with good dissolution properties.

### BRIEF DESCRIPTION OF THE DRAWINGS

Fig. 1 is a graph showing the dissolution profile of example 1.
Fig. 2 is a graph showing the dissolution profile of example 2.
Fig. 3 is a graph showing the dissolution profile of example 3.
Fig. 4 is a graph showing the dissolution profile of comparative example 1.

### BRIEF SUMMARY OF THE INVENTION

The inventors of the present invention have surprisingly found that a tablet formulation comprising edoxaban or a pharmaceutically acceptable salt thereof, a first diluent and a second diluent, wherein said second diluent is different from said first diluent, have an adequate dissolution profile, when compared with other tablet formulations known in the art.

A second aspect of the present invention refers to a process for the preparation of a tablet formulation according to the first aspect.

### DETAILED DESCRIPTION OF THE INVENTION

The inventors have surprisingly found that a tablet comprising edoxaban or a pharmaceutical acceptable salt thereof, a lactose, as a first diluent and a starch, as a second diluent in a weight ratio equal or greater than 2.5:1, shows a good to excellent dissolution profile. This comes unexpectedly, since other compositions of the prior art comprising edoxaban and two diluents have unacceptable dissolution rates, requiring more than 30 minutes to dissolve 85% of the active ingredient, whereas the generally desired dissolution rate is 90% in less than 15 minutes.

Thus, in a first aspect, the present invention refers to a tablet composition comprising edoxaban or a pharmaceutically acceptable salt thereof, a first diluent and a second diluent, wherein said second diluent is different from said first diluent.

In an embodiment, the present invention refers to a tablet composition comprising edoxaban or a pharmaceutically acceptable salt thereof, a lactose as a first diluent, and a starch as a second diluent.

In an embodiment, the weight ratio of lactose, as first diluent to starch, as second diluent, in the tablets according to the present invention is equal or greater than 2.5:1 (lactose:starch), preferably equal or greater than 2.7:1, more preferably equal or greater than 2.9:1, more preferably equal or greater than 3:1, even more preferably equal or greater than 3.2:1, even more preferably equal or greater than 3.3:1, even more preferably equal or greater than 3.5:1, even more preferably equal or greater than 3.7:1 and even more preferably equal or greater than 4:1 (lactose: starch).

In an embodiment, the weight ratio of lactose, as a first diluent, to starch, as a second diluent, in the tablets according to the present invention is comprised between 2.5:1 and 20:1 (lactose:starch), preferably between 2.5:1 and 10:1, more preferably between 2.7:1 and 10:1, more preferably between 2.9:1 and 10:1, even more preferably between 3:1 and 10:1, even more preferably between 3.2:1 and 10:1, even more preferably between 3.3:1 and 10:1, even more preferably between 3.3:1 and 8:1, even more preferably between 3.5:1 and 8:1, even more preferably between 3.7:1 and 8:1, and even more preferably between 4:1 and 8:1.

In a particular embodiment, the tablet of the present invention comprises edoxaban or a pharmaceutically acceptable salt thereof, a lactose as a first diluent, and a starch as a second diluent, wherein the starch is pregelatinized starch, and wherein the weight ratio of a lactose to pregelatinized starch is about 4:1. Preferably, the lactose is lactose monohydrate.

In an embodiment the tablets according to the first aspect do not comprise sugar alcohols. Sugar alcohols are known to cause intolerances and may result in lower therapeutic efficacy.

In another embodiment the tablets according to the first aspect do not comprise soluble vinylpyrrolidone polymers, such as povidone and copovidone due to their high peroxide content degrade a pharmaceutical composition over time.

In a particular embodiment, the tablets according to the invention comprise lactose and starch as the sole diluents.

In a particular embodiment, the tablets according to the invention do not comprise sugar alcohols and do not comprise soluble vinylpyrrolidone polymers.

In an embodiment the tablets according to the first aspect comprise lactose and starch but do not comprise sugar alcohols.

In another embodiment the tablets according to the first aspect comprise lactose and starch but do not comprise soluble vinylpyrrolidone polymers, such as povidone and copovidone.

In an embodiment the tablets according to the first aspect comprise lactose, as a first diluent, and a starch, as a second diluent, in a weight ratio lactose: starch of equal or greater than 2.5:1, preferably equal or greater than 2.7:1, more preferably equal or greater than 2.9:1, more preferably equal or greater than 3:1, even more preferably equal or greater than 3.2:1, even more preferably equal or greater than 3.3:1, even more preferably equal or greater than 3.5:1, even more preferably equal or greater than 3.7:1 and even more preferably equal or greater than 4:1; with one or more of the following provisos: i) the tablets do not comprise sugar alcohols; ii) the tablets do not comprise water soluble vinylpyrrolidone polymers, such as povidone and copovidone; iii) the tablets contain lactose and starch as the two sole diluents; and iv) the tablets contain only one binder

Further, the tablets according to the first aspect comprise one or more binders. Preferably, only one binder is used, for example hydroxypropyl cellulose, and not two or more. This results in pharmaceutical compositions requiring less ingredients, which results in less complicated compositions that can be manufactured at lower costs.

In the context of the present invention, an *"adequate dissolution profile"* refers to tablets having a dissolution profile, wherein at least about 90% of the active ingredient dissolves in the test media, in less than 15 minutes, preferably in less than 10 minutes, more preferably in less than 5 minutes. Preferably, the tablets have a dissolution profile wherein at least 90% of the active ingredient dissolves in the test media, in less than 15 minutes, preferably in less than 10 minutes, more preferably in less than 5 minutes.

The method used for obtaining dissolution profiles according to the present invention is as follows: A paddle dissolution test is carried out, wherein a tablet is added to 900 mL of acetate buffer at pH 4.0 at 37 ± 0.5°C with a rotation speed of 50 rpm. Samples are taken every 5 minutes.

In the context of the present invention, *"edoxaban"* is used to encompass both the free base *N*'-(5-chloropyridin-2-yl)-*N*-[(1*S*,2*R*,4*S*)-4-(dimethylcarbamoyl)-2-[(5-methyl-6,7-dihydro-4*H*-[1,3]thiazolo[5,4-c]pyridine-2-carbonyl)amino]cyclohexyl]oxamide as well as the pharmaceutically acceptable salts thereof. In a preferred embodiment edoxaban is used in the form of its tosylate salt, more preferably in the form of the monohydrate of said tosylate salt.

In an embodiment the tablet according to present invention further comprises additional pharmaceutically acceptable excipients selected from the group comprising diluents, binder, disintegrants, lubricants, and optionally colorants, sweeteners or aromas.

Non-limiting examples of a suitable lactose for the tablets according to the present invention are: alfa-lactose, beta-lactose, lactose monohydrate, anhydrous lactose, and/or mixtures thereof. Preferably, the lactose is lactose monohydrate. The suitable lactose may be comprised in the tablet in an amount of 50 to 75 % by weight, preferably 50 to 70 % by weight, and more preferably 55 to 70 % by weight.

Non-limiting examples of suitable starches for the tablets according to the present invention are: cornstarch, potato starch, rice starch, pregelatinized starch, semi-digested starch, and/or mixtures thereof. Preferably, the starch is pregelatinized starch. The suitable starch may be comprised in the tablet in an amount of 6.0 to 25 % by weight, preferably 6.0 to 21 % by weight, and more preferably 6.0 to 17 % by weight.

Non-limiting examples of other suitable diluents, if present in addition to the first and second diluents in the tablets of the present invention, are calcium phosphate, calcium hydrogen phosphate, di-calcium phosphate hydrate and/or mixtures thereof, with the proviso that said additional diluent is only used, when no povidone and no copovidone is present in the composition. The suitable other diluent may be comprised in the tablet in an amount of 0 to 25 % by weight.

Non-limiting examples of suitable binders for the tablets of the present invention are microcrystalline cellulose, hydroxypropyl cellulose, sodium alginate, chitosan, hydroxypropyl methylcellulose, hydroxyethyl cellulose, sodium carboxymethyl cellulose, carboxymethylhydroxyethyl cellulose and/or mixtures thereof. Preferably the binder is hydroxypropyl cellulose. The suitable binder(s) may be comprised in the tablet in an amount of 0.5 to 7 % by weight, preferably 1.5 to 5 % by weight, and more preferably 2.5 to 5 % by weight.

Non-limiting examples of suitable disintegrants for the tablets of the present invention are sodium starch glycolate, calcium silicate, sodium croscarmellose, and/or mixtures thereof. Preferably, the disintegrant is sodium starch glycolate. Sodium starch glycolate is not considered being a diluent, and thus is not a starch as defined above. The suitable disintegrant(s) may be comprised in the tablet in an amount of 0.1 to 8 % by weight, preferably 0.5 to 7 % by weight, and more preferably 1 to 5 % by weight.

Non-limiting examples of suitable lubricants for the tablets of the present invention are sodium benzoate, stearic acid, magnesium stearate, calcium stearate, zinc stearate, sodium stearyl fumarate, glyceryl behenate, glyceryl monostearate, and/or mixtures thereof. Preferably, the lubricant is magnesium stearate. The suitable lubricant(s) may be comprised in the tablet in an amount of 0.05 to 2 % by weight, preferably 0.1 to 2 % by weight, and more preferably 0.5 to 1.5 % by weight.

In an embodiment the tablet according to present invention comprises:
a. 1 to 30% in weight of the tablet of edoxaban or a pharmaceutically acceptable salt; and
b. 50 to 80% in weight of the tablet of a combination of a first diluent and a second diluent, with the proviso that the weight ratio of the first diluent to the second diluent is equal or greater than 2.5:1, preferably equal or greater than 2.7:1, more preferably equal or greater than 2.9:1, more preferably equal or greater than 3:1, even more preferably equal or greater than 3.2:1, even more preferably equal or greater than 3.3:1, even more preferably equal or greater than 3.5:1, even more preferably equal or greater than 3.7:1 and even more preferably equal or greater than 4:1;
with one or more of the following further provisos: i) the tablets do not comprise sugar alcohols; ii) the tablets do not comprise water soluble vinylpyrrolidone polymers, such as povidone and copovidone; iii) the tablets contain lactose and starch as the two sole diluents; and iv) the tablets contain only one binder.

In an embodiment the tablet according to present invention comprises:
a. 1 to 30% in weight of the tablet of edoxaban or a pharmaceutically acceptable salt;
b. 50 to 75% in weight of the tablet of lactose; and
c. 6.0 to 25 % in weight of the tablet of a starch.

In a particular embodiment the tablet according to present invention comprises:
a. 1 to 30% in weight of the tablet of edoxaban or a pharmaceutically acceptable salt;
b. 50 to 75% in weight of the tablet of lactose;
c. 6.0 to 25 % in weight of the tablet of a starch;
d. 0.5 to 7% in weight of the tablet of a binder;
e. 0.1 to 8% in weight of the tablet of a disintegrant;
f. 0.05 to 2% in weight of the tablet of a lubricant.

In a particular embodiment lactose is lactose monohydrate, the starch is pregelatinized starch, the binder is hydroxypropyl cellulose, the disintegrant is sodium starch glycolate, and the lubricant is magnesium stearate.

In an embodiment the tablet according to present invention, further comprises a coating. Non-limiting examples of coatings comprise coating agents containing for example poly(vinyl alcohol) (PVA), hydroxypropyl methylcellulose (HPMC) or polyethyleneglycol-poly(vinyl alcohol) (PEG-PVA) graft copolymer. For example, immediate release type coatings containing a polymer, a plasticizer and a pigment in a dry concentrate, such as Opadry® from Colorcon may be used.

The invention further relates to the tablets according to the present invention for use as a medicament in the prevention and/or treatment of thrombosis or embolism.

The invention also relates to the use of tablets according to the present invention in the manufacture of a medicament for the prevention and/or treatment of thrombosis or embolism.

The invention also relates to a method of prevention and/or treatment of thrombosis or embolism, comprising administering to said subject a tablet according to the present invention.

In a second aspect the present invention relates to a process for the preparation of the tablets according to the first aspect, comprising:
i. preparing a mixture comprising edoxaban or a pharmaceutically acceptable salt thereof, a first diluent, and a second diluent, a first portion of a binder (if present), and a first portion of a disintegrant (if present); and
ii. converting the mixture of step i) into a tablet.

Preferably, the process for the preparation of the tablets according to the first aspect, comprises the steps of:
i. preparing a mixture comprising edoxaban or a pharmaceutically acceptable salt thereof, first diluent, a second diluent, a first portion of the binder (if present), and a first portion of the disintegrant (if present); wherein the first diluent is a lactose, and wherein the second diluent is a starch; and
ii. converting the mixture of step i) into a tablet.

Preferably, the process for the preparation of the tablets according to the first aspect, comprises the steps of:
i. preparing a mixture comprising edoxaban or a pharmaceutically acceptable salt thereof, a first diluent, a second diluent, a first portion of the binder (if present), and a first portion of the disintegrant (if present); wherein the first diluent is a lactose, and wherein the second diluent is a starch; and
ii. converting the mixture of step i) into a tablet; wherein step ii) comprises granulating the mixture resulting from step i) using either wet granulation or dry granulation.

Preferably, the process for the preparation of the tablets according to the first aspect wherein the tablets comprise a binder, comprises the steps of:
i. preparing a mixture comprising edoxaban or a pharmaceutically acceptable salt thereof, first diluent, a second diluent, a first portion of the binder, and a first portion of the disintegrant (if present); wherein the first diluent is a lactose, and wherein the second diluent is a starch; and
ii. wet-granulating the mixture resulting from step i); with an aqueous solution comprising the remaining amount (second portion) of the binder.

More preferably, the process for the preparation of tablets comprising a binder and a disintegrant comprises the steps of:
i. preparing a mixture comprising edoxaban or a pharmaceutically acceptable salt thereof, a lactose (as a first diluent), a starch (as a second diluent), a first portion of the binder, and a first portion of the disintegrant;
ii. wet-granulating the mixture resulting from step i); with an aqueous solution comprising the remaining amount (second portion) of the binder;
iii. drying the granules resulting from of step ii);
iv. milling the granules resulting from step iii);
v. mixing the product resulting from step iv) with the remaining amount (second portion) of the disintegrant.

More preferably, the above mentioned process further comprises:
i. preparing a mixture comprising edoxaban or a pharmaceutically acceptable salt thereof, a lactose (as a first diluent), a starch (as a second diluent), a first portion of the binder, and a first portion of the disintegrant;
ii. wet-granulating the mixture resulting from step i); with an aqueous solution comprising the remaining amount (second portion) of the binder;
iii. drying the granules resulting from of step ii);
iv. milling the granules resulting from step iii);
v. mixing the product resulting from step iv) with the remaining amount (second portion) of the disintegrant;
vi. adding a lubricant to the mixture of step v);
vii. compressing the mixture resulting from step vi); and optionally,
viii. coating the tablets obtained in step vii).

In a preferred embodiment, lactose is lactose monohydrate, the starch is pregelatinized starch, the binder is hydroxypropyl cellulose, the disintegrant is sodium starch glycolate, and the lubricant is magnesium stearate.

In a preferred embodiment, the first diluent, preferably a lactose, is added in two separate portions. In particular, the preparation of the mixture of step i) is carried in two steps; in the first step edoxaban, a first portion of the first diluent, the second diluent, a first portion of the binder, and a first portion of the disintegrant are mixed to obtain a first mixture and in a second step the rest (second portion) of the first diluent is added to the first mixture and further mixed to obtain a second mixture. The first portion of the first diluent is added to the mixer as the first material of the mixture and the second portion of the first diluent is added as the last material of the mixture. This is known as the "sandwich method" and has the advantage that the resulting mixture shows good homogeneity of the blend after the step of mixing.

### Examples

### Dissolution test conditions:

- Paddle dissolution test.
- Rotation Speed: 50 rpm.
- Dissolution media: Acetate buffer pH 4.0
- Dissolution volume: 900 mL.
- Sample volume: 10 mL
- Tablets per well: 1 tablet
- Temperature: 37 ± 0.5°C.
- Extraction times: a sample was taken at 5, 10, 15, 20, 30 and 45 min (with replenishment of volume). After the sample at 45 minutes has been taken, rotation speed was increased to 150 rpm for ensuring complete dissolution, and a last sample was taken at 55 minutes.

### Chromatographic conditions:

- Column: ACQUITY™UPLC™ CSH C18, 50 x 2.1 mm, 1.7 µm
- Mobile phase: Methanol / Ammonium bicarbonate buffer 10 mM pH 8.2, Isocratic (50/50)
- Flow: 0.6 mL/min.
- Column temperature: 45°C
- Sample temperature: 15°C
- Detection wavelength: 290 nm
- Injection volume: 2 µL (Loop 2 µL - Full loop)

**Table 1**

| | Example 1 | | Example 2 | | Example 3 | |
|---|---|---|---|---|---|---|
| Material | Weight per Tablet (mg) | % by weight | Weight per Tablet (mg) | % by weight | Weight per Tablet (mg) | % by weight |
| Edoxaban tosylate monohydrate | 40.41 | 19.34 | 40.41 | 19.34 | 40.41 | 19.34 |
| Lactose monohydrate | 120.99 | 57.89 | 107.86 | 51.61 | 137.27 | 65.68 |
| Pregelatinized starch | 30.00 | 14.35 | 43.14 | 20.64 | 13.73 | 6.57 |
| Hydroxypropyl cellulose | 7.54 | 3.61 | 7.53 | 3.60 | 7.53 | 3.60 |
| Na Starch glycolate | 8.36 | 4.00 | 8.36 | 4.00 | 8.36 | 4.00 |
| Magnesium stearate | 1.70 | 0.81 | 1.70 | 0.81 | 1.70 | 0.81 |
| TOTAL | 209.00 | 100 | 209.00 | 100 | 209.00 | 100 |

The tablets, having the above-mentioned composition, have been prepared following the process described below, specified with the quantities of example 1.

40.41 mg of edoxaban tosylate monohydrate are mixed in a double cone blender operated at 18 rpm during 25 minutes with 60.495 mg of lactose monohydrate (first portion of first diluent), 30.00 mg of pregelatinized starch, 2.56 mg of hydroxypropyl cellulose (first portion of binder), 2.09 mg of sodium starch glycolate (first portion of disintegrant) and 60.495 mg of lactose monohydrate (second portion of first diluent). The mixture is then wet-granulated in a fluid bed at 30°C with an aqueous solution comprising 4.98 mg of hydroxypropyl cellulose (second portion of binder) in 7.54 mg of water. The granules are then dried in a fluid bed and milled in a conical mill at 1750 rpm using a 0.9 mm sieve. The resulting powder is mixed with 6.27 mg of sodium starch glycolate (second portion of disintegrant) in a double cone blender operated at 18 rpm for 15 minutes. Then, 1.70 mg of magnesium stearate are added to the blender which is further operated at 18 rpm for 5 minutes and the resulting mixture is tabletted in a rotary tabletting press operated at 90 N and a speed of 50 tablets/minutes. The tablets were then coated with Opadry® 03F220132 Yellow in a fully perforated drum.

Examples 2 and 3 were manufactured in the same way, except for using the quantities as shown in table 1 and both examples 2 and 3 were not coated.

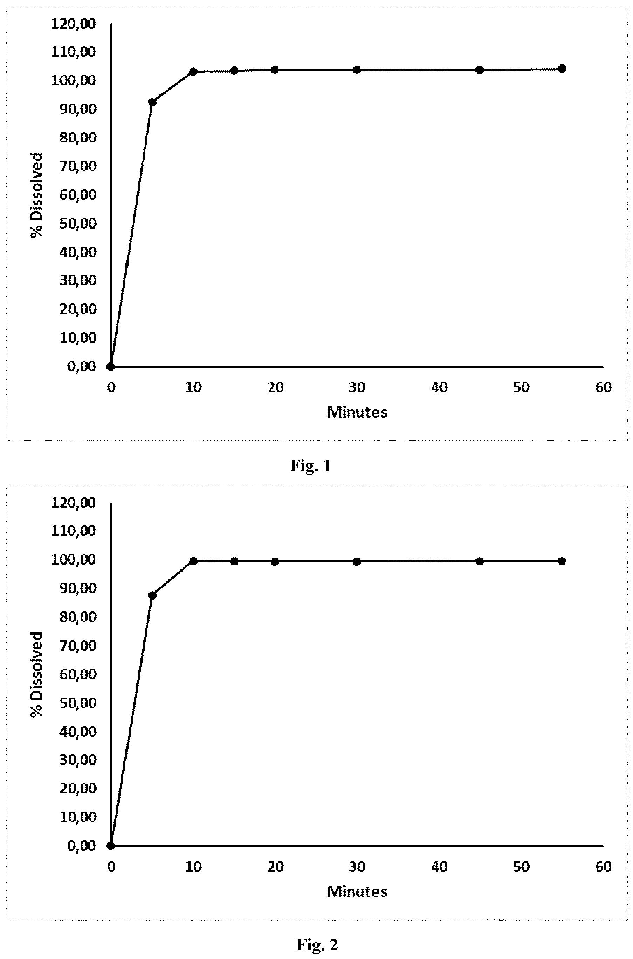

As it can be seen in figures 1, 2 and 3, dissolution profiles of examples 1, 2 and 3, having a lactose:starch ratio greater than 2.5:1, have good to excellent dissolution properties.

Comparative example 1 has been carried out following experiment C from patent document EP 2 140 867 A1, as follows: ingredients shown in Table 2, excepting hydroxypropyl cellulose and magnesium stearate, were mixed by means of a mortar, and the mixture was granulated by use of aqueous hydroxypropyl cellulose solution. The thus-produced granules were mixed with magnesium stearate, to thereby yield granules were compressed into tablets using round shaped dies with a diameter of 8.0mm and punches at 7.8 kN of compression force, to thereby yield tablets of interest.

**Table 2**

| | |
|---|---|
| Compound 1 (Edoxaban) | 40.41 |
| Lactose | 99.79 |
| Pregelatinized starch | 42.8 |
| Sodium starch glycolate | 10.0 |
| Hydroxypropylcellullose | 6.0 |
| Mg stearate | 1.0 |
| Weight tablet | 200.0 mg |

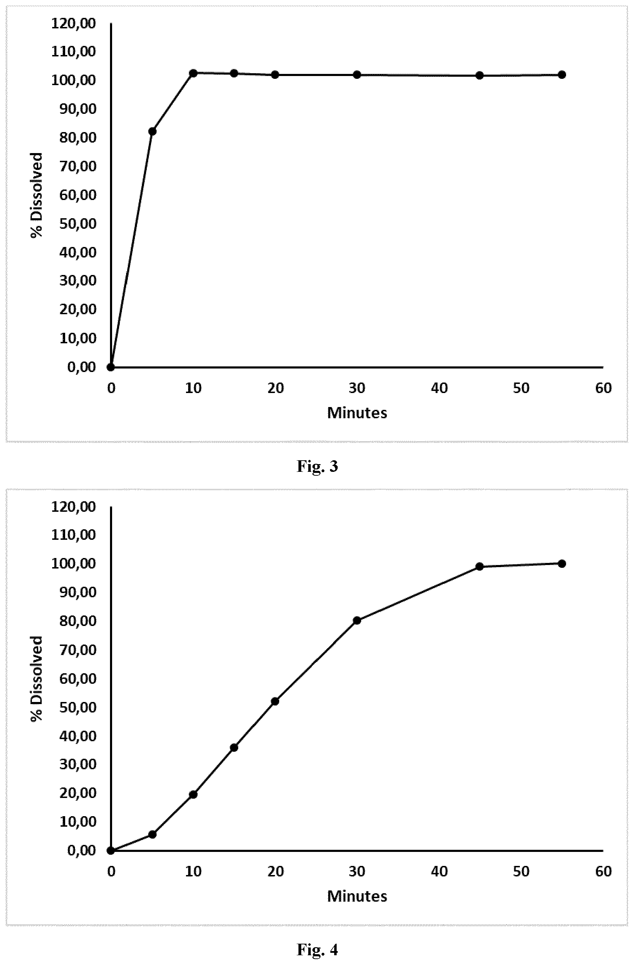

As can be seen in figure 4, this composition does not have good dissolution properties, having a more slow dissolution profile.

## Claims

1. A tablet comprising edoxaban or a pharmaceutically acceptable salt thereof, a lactose, as a first diluent and a starch, as a second diluent.

2. A tablet according to claim 1, wherein the weight ratio of lactose to starch is equal or greater than 2.5:1.

3. The tablet according to any one of claims 1 or 2, wherein the tablet does not comprise at least one of a) sugar alcohols and b) soluble vinylpyrrolidone polymers.

4. A tablet according to any one of claims 1 to 3, wherein the weight ratio of lactose to a starch is comprised between 2.5:1 and 20:1, preferably between 2.5:1 and 10:1, more preferably between 2.7:1 and 10:1, more preferably between 2.9:1 and 10:1, even more preferably between 3.1:1 and 10:1, even more preferably between 3.3:1 and 10:1, even more preferably between 3.3:1 and 8:1, and even more preferably between 4:1 and 8:1.

5. A tablet according to any one of claims 1 to 4, wherein the lactose is selected from the group comprising alfa-lactose, beta-lactose, lactose monohydrate, anhydrous lactose, and/or mixtures thereof, preferably lactose monohydrate.

6. A tablet according to any one of claims 1 to 5, wherein the starch is selected from the group comprising cornstarch, potato starch, rice starch, pregelatinized starch, semi-digested starch, and/or mixtures thereof, preferably pregelatinized starch.

7. A tablet according to any one of the preceding claims, further comprising additional pharmaceutically acceptable excipients selected from the group comprising binders, disintegrants, lubricants, and optionally colorants, sweeteners or aromas.

8. A tablet according to claim 7, comprising a third diluent in addition to lactose and starch, wherein the third diluent is selected from the group comprising calcium phosphate, calcium hydrogen phosphate or di-calcium phosphate hydrate and/or mixtures thereof, with the proviso that the tablet does not simultaneously contain povidone and copovidone.

9. A tablet according to any of claims 7 to 8, wherein the binder is selected from the group comprising microcrystalline cellulose, hydroxypropyl cellulose, hydroxypropyl methylcellulose, hydroxyethyl cellulose, sodium carboxymethyl cellulose, carboxymethylhydroxyethyl cellulose, and/or mixtures thereof, preferably hydroxypropyl cellulose.

10. A tablet according to any one of claims 7 to 9, wherein the disintegrant is selected from the group comprising sodium starch glycolate, calcium silicate, sodium croscarmellose and/or mixtures thereof, preferably sodium starch glycolate.

11. A tablet according to any one of claims 7 to 10, wherein the lubricant is selected from the group comprising sodium benzoate, stearic acid, magnesium stearate, calcium stearate, zinc stearate, sodium stearyl fumarate, glyceryl behenate, glyceryl monostearate, and/or mixtures thereof, preferably magnesium stearate.

12. A tablet according to claim 11, comprising lactose monohydrate, pregelatinized starch, hydroxypropyl cellulose, sodium starch glycolate and magnesium stearate.

13. A tablet according to claims 1 to 11, wherein the tablet comprises:
a) 1 to 30% in weight of the tablet of edoxaban or a pharmaceutically acceptable salt;
b) 50 to 75% in weight of the tablet of lactose;
c) 6.0 to 25 % in weight of the tablet of a starch;
d) 0.5 to 7% in weight of the tablet of a binder;
e) 0.1 to 8% in weight of the tablet of a disintegrant; and
f) 0.05 to 2% in weight of the tablet of a lubricant.

14. A process for the preparation of a tablet as defined in anyone of claims 1 to 13, wherein the tablet comprises a binder and a disintegrant, comprising:
i. preparing a mixture comprising edoxaban or a pharmaceutically acceptable salt thereof, lactose, a starch, a first portion of the binder, and a first portion of the disintegrant; and
ii. converting the mixture of step i) into a tablet.

15. The process according to claim 14, wherein step ii) comprises granulating the mixture resulting from step i) using either wet granulation or dry granulation.
